# EUROPEAN PATENT APPLICATION

(11) **EP 4 454 555 A1**
(43) Date of publication of application: **30.10.2024**
(21) Application number: 24172191.9
(22) Date of filing: 24.04.2024
(51) Int. Cl.: A61B 5/145, A61B 5/1473, A61B 5/1486, A61B 5/1495, A61B 5/00

(54) **GLUCOSE SENSOR WITH IN VIVO ELECTRODE CLEANING**

(30) Priority: 27.04.2023 US 202363498717 P; 27.03.2024 US 202418618694
(71) Applicant: Medtronic MiniMed, Inc., Northridge, CA 91325 (US)
(72) Inventor: Rais, Nor Akmaliza, Northridge, 91325 (US); Chiu, Chia-Hung, Northridge, 91325 (US); Buganski, Meredith, Northridge, 91325 (US); Mahadevan, Aishwarya, Northridge, 91325 (US)
(74) Representative: Zimmermann & Partner Patentanwälte mbB

(57) **Abstract**

A device includes a glucose monitor comprising at least one sensor electrode configured to sense signals indicative of interstitial glucose level of a patient. The device also includes a memory and one or more processors implemented in circuitry and in communication with the memory. The one or more processors are configured to cause delivery of a cleaning electrical current to the at least one sensor electrode. The cleaning electrical current is one or more electrochemical cleaning pulses configured to at least partially remove, in vivo and from the sensor electrode, excipients associated with the delivery of a fluid that includes insulin.

## Description

This application claims the benefit of U.S. Provisional Patent Application No. 63/498,717, filed 27 April 2023, the entire contents of which is incorporated herein by reference.

### TECHNICAL FIELD

This disclosure relates to glucose sensors.

### BACKGROUND

Glucose sensors are configured to detect and/or quantify the amount of glucose in a patient's body (e.g., interstitial glucose or possibly blood glucose), which enables patients and medical personnel to monitor physiological conditions within the patient's body. In some examples, it may be beneficial to monitor glucose levels on a continuing basis (e.g., in a diabetic patient). Thus, glucose sensors have been developed for use in obtaining an indication of glucose levels in a diabetic patient. Such indications are useful in monitoring and/or adjusting a treatment regimen, which typically includes administration of insulin to the patient.

A patient can measure their blood glucose (BG) using a BG measurement device (i.e., glucose meter), such as a test strip meter. A continuous glucose measurement system (or a continuous glucose monitor (CGM)) may be configured to determine interstitial glucose; although possible for CGM to determine blood glucose. A hospital hemocue may also be used to determine glucose level. CGMs may be beneficial for patients who desire to take more frequent glucose measurements. Some example CGM systems include subcutaneous (or short-term) sensors and implantable (or long-term) sensors.

### SUMMARY

In general, this disclosure describes techniques for electrochemical cleaning of glucose sensors (e.g., a continuous glucose monitor or "CGM"). In some examples, the electrochemical cleaning may be accomplished by application of one or more pulses of electrical current to a sensor electrode of a glucose monitor, which may at least partially remove excipients (e.g., insulin excipients such as phenol, m-cresol, and./or glycerin) associated with delivery of insulin. For instance, this disclosure describes techniques and devices for addressing "electrode poisoning" associated with "two-in-one" or "all-in-one" diabetes management devices which may both monitor glucose with a CGM and deliver insulin in close proximity to the glucose monitor. Although described primarily with respect to devices that both monitor glucose and deliver insulin, it should be understood that the described techniques and devices may include a discreet glucose monitor and a separate discreet insulin delivery device.

In one example, a device includes a glucose monitor comprising at least one sensor electrode configured to sense signals indicative of interstitial glucose level of a patient. The device also includes a memory and one or more processors implemented in circuitry and in communication with the memory. The one or more processors are configured to cause delivery of a cleaning electrical current to the at least one sensor electrode, the cleaning electrical current being one or more electrochemical cleaning pulses configured to at least partially remove, in vivo and from the sensor electrode, excipients associated with the delivery of a fluid that includes insulin.

In another example, a technique includes determining, via one or processors implemented in circuitry and in communication with a memory of a device that includes a glucose monitor, that a cleaning electrical current should be delivered to at least one sensor electrode of the glucose monitor. The at least one sensor electrode is configured to sense signals indicative of the interstitial glucose level of a patient. The technique also includes causing delivery of the cleaning electrical current to the at least one sensor electrode. The cleaning electrical current is one or more electrochemical cleaning pulses configured to at least partially remove, in vivo and from the sensor electrode, excipients associated with the delivery of a fluid that includes insulin.

In another example, a non-transitory computer-readable storage medium includes instructions that, when executed, configure a processor to determine, via one or processors implemented in circuitry and in communication with a memory of a device that includes a glucose monitor, that a cleaning electrical current should be delivered to at least one sensor electrode of the glucose monitor. The at least one sensor electrode is configured to sense signals indicative of the interstitial glucose level of a patient. The instructions cause delivery of the cleaning electrical current to the at least one sensor electrode. The cleaning electrical current is one or more electrochemical cleaning pulses configured to at least partially remove, in vivo and from the sensor electrode, excipients associated with the delivery of a fluid that includes insulin.

Further disclosed herein is a device that includes a glucose monitor comprising at least one sensor electrode configured to sense signals indicative of interstitial glucose level of a patient. The device also includes a memory and one or more processors implemented in circuitry and in communication with the memory. The one or more processors are configured to cause delivery of a cleaning electrical current to the at least one sensor electrode. The cleaning electrical current is one or more electrochemical cleaning pulses configured to at least partially remove, in vivo and from the sensor electrode, excipients associated with the delivery of a fluid that includes insulin.

The details of one or more examples are set forth in the accompanying drawings and the description below. Other features, objects, and advantages will be apparent from the description and drawings, and from the claims.

### BRIEF DESCRIPTION OF DRAWINGS

FIG. 1 is a schematic diagram illustrating an example glucose level management system, in accordance with one or more examples described in this disclosure.
FIG. 2 is a side view of an example medical device including a glucose monitor and insulin cannula.
FIG. 3 is a conceptual cross-sectional view of an example medical device including a glucose monitor and insulin cannula.
FIG. 4 is a conceptual side-view of an example medical device including a glucose monitor and insulin cannula.
FIG. 5 is a block diagram illustrating an example glucose sensor, in accordance with one or more examples described in this disclosure.
FIG. 6 is a schematic diagram illustrating example sensor electrodes and a voltage being applied to the sensor electrodes.
FIGS. 7A-7C are schematic diagrams illustrating the buildup of a thin film on an example electrode of an electrochemical cell due to insulin excipients.
FIG. 8 is a schematic diagram illustrating the example electrochemical interference due to insulin excipients on an example electrode of an electrochemical cell.
FIG. 9A is a conceptual chart illustrating an example cleaning pulse on an example poisoned electrode.
FIG. 9B is a chart of an example interference test on an example electrode.
FIG. 10 is a schematic diagram illustrating an example polymerization pathway of an insulin excipient.
FIG. 11 is a chart illustrating an example electrochemical pulse using a cyclic voltammetry technique.
FIGS. 12A-12E are examples charts illustrating the cleaning effect of a sweeping cleaning pulse of electrical current.
FIGS. 13A-13D are schematic illustrations of example waveforms of an electrochemical cleaning pulse.
FIG. 14 is a schematic illustration of example waveform of an electrochemical cleaning pulse.
FIGS. 15 are charts illustrating the observed response of an example sensor before and after an electrochemical cleaning pulse.
FIG. 16 illustrates the observed response of an example sensor after an electrochemical cleaning pulse.
FIG. 17 illustrates the observed response of an example sensor after an electrochemical cleaning pulse.
FIG. 18 is a flowchart illustrating an example technique for electrochemical cleaning.

### DETAILED DESCRIPTION

Diabetes patients who use sensor-augmented pump (SAP) therapy may do so by inserting an infusion set and a continuous glucose monitoring (CGM) sensor (e.g., glucose monitor) separately. The burden of two insertions increases the likelihood of adverse effects of skin penetration, reaction to adhesives, and site loss. Combining the insulin delivery with glucose monitoring into a single integrated device addresses these concerns. However, a glucose monitor in close proximity to an infusion cannula may experience electrochemical interference. For example, fluid delivered through an infusion set may include one or more excipients and insulin, which may act as interference on a sensor electrode of the glucose monitor. In some examples, the excipients may polymerize and/or buildup on the sensor electrode, blocking the sensor electrode from accurately monitoring current used for determining glucose level. As such, electrochemical interference can cause the glucose monitor to provide an inaccurate glucose reading, which may lead to less optimal insulin dosage computation. This phenomenon may be referred to as "electrode poisoning." Films or buildup of insulin excipients on a sensor electrode may be addressed by removal of the glucose monitor from the patient and physically cleaning or polishing the sensor electrode. Removal of all or part of the device may be impractical or undesirable in some instances. Example devices and techniques disclosed herein may at least partially remove excipients associated with the delivery of insulin by electrochemically cleaning the sensor electrode via delivery of an electrical current to the sensor electrode.

A glucose sensor may include an electrochemical cell which may be used to determine a glucose level of a patient. As one example, the glucose sensor may determine interstitial glucose level. The electrochemical cell may apply electrical energy via one or more sensor electrodes displaced within a fluid and/or tissue of a patient, and based on the impedance, determine the glucose level. However, the one or more sensor electrodes may be susceptible to materials within the infused fluid in addition to the insulin, which may be a commercially available insulin formulation. These additional materials are referred to herein as insulin excipients or simply excipients. A bias voltage applied to the sensor electrodes may be needed for the electrodes to function properly. In the presence this bias voltage, the electrodes may cause oxidation of one or more of the substances within the infused fluid. With sufficient prolonged exposure to the substances, the oxidation can lead to deposits and build-up of a thin film of insulin excipients or their reaction products on a surface of a sensor electrode. When the interfering substances are oxidized, they can add or subtract from the signal current coming from the glucose, which can lead to inaccurate glucose readings. With prolonged exposure to the substances, the thin film formed on an electrode can cause a permanent change in response characteristics to the glucose. That is, a change in the electrical response of the electrochemical cell to the presence of an analyte, such as glucose. The materials that deposit and form the thin film may be excipients of insulin, e.g., m-cresol, phenol, glycerin, or other materials. In some examples, oxidation of interfering compounds alone leads to a temporary interference while thin film deposits on an electrode lead to a permanent change to the electrochemical response of the sensor.

In some examples, a glucose sensor, such as a CGM, and an insulin delivery device, such as an insulin pump, may be packaged together (e.g., in a "two-in one" device, also called an "all-in-one" device) such that the electrode of the glucose sensor may be placed relatively near an insulin delivery site on the patient. Relatively near may mean that the glucose sensor and the insulin delivery site are within about 10 centimeters from each other, or within about 5 centimeters from each other, or within about 2 centimeters from each other. In some examples, the glucose sensor and insulin cannula may be configured to be inserted by a single needle, which may reduce or minimize pain, discomfort, and risk to the patient. In such examples, the insulin cannula may be deeper (e.g., further under the patient's skin) than the glucose sensor, or the glucose sensor may be deeper than the insulin cannula.

Insulin excipient concentration may be greatest nearest the insulin delivery site and may decrease as the excipients diffuse within the patient. In some examples, the higher the concentration of the interfering compounds, the higher the rate of oxidation, and the faster the buildup of a thin film on a glucose sensor electrode causing an increased electrochemical interference. In some examples, even low concentrations of excipients and/or interfering compounds may cause interference followed by a buildup of a thin film on a glucose sensor electrode, e.g., over time, causing progressively increasing electrochemical interference.

The thin film buildup of insulin excipients on a sensor electrode may change the dielectric properties of the electrode and the sensor electrical circuit system, thereby causing the electrochemical interference. Electrochemical interference may confound glucose measurement based on an electrical signal. For example, a glucose monitor may use a signal electrical current, or signal current, to determine the presence and/or amount of glucose at or near an electrode. Changes in the signal current may be caused by increasing and/or decreasing glucose, electrochemical interference, or both.

In one or more examples, a device may include both a glucose monitor which includes at least one sensor electrode and an insulin infusion cannula, and thus be configured to both measure and monitor an interstitial glucose level of the patient and deliver insulin to the patient. The device may also include a memory and one or more processors implemented in circuitry. The one or more processors may be configured to cause delivery of a cleaning electrical current to the at least one sensor electrode. The cleaning electrical current may be one or more electrochemical cleaning pulses configured to at least partially remove, in vivo and from the sensor electrode, excipients associated with the delivery of a fluid that includes insulin. In some examples, the one or more processors may be configured to monitor a signal electrical current as the signal current of the glucose monitor. The one or more processors may also be configured to output an output signal that causes a cleaning electrical current to be delivered to a sensor electrode of the glucose monitor. In this way, the buildup of insulin excipients on a sensor electrode may be addressed by cleaning the sensor electrode in vivo (i.e., within the patient's body), which may reduce or eliminate the need to remove the device for cleaning associated with insulin excipient buildup.

The cleaning electrical current may be one or more current pulses. The current pulse or pulses may be configured in a number of ways. For example, the one or more processors may be configured to cause delivery of the cleaning current responsive to a determination that the impedance at the glucose monitor satisfies an impedance threshold or a threshold rate of impedance change, or a signal electrical current from the glucose monitor satisfies a current threshold or satisfies a threshold rate of current change. For example, the insulin pump of the device may be configured to deliver basal dosages, which are small amounts of insulin released continuously throughout the day, and bolus dosages of insulin, which are larger dosages associated with a glucose level increase, such as due to eating. The delivery of a bolus dose may cause the signal electrical current to satisfy a threshold or a threshold rate of change. In this way, the electrochemical cleaning pulse(s) may be delivered relative to delivery of bolus dosages, such as following delivery of a bolus dose. In some examples, a time delay may be built in, such that delivery of the cleaning electrical current is scheduled 1 minute, 2 minutes, 5 minutes, 10 minutes, or the like after the signal electrical current satisfies the threshold. In this way, insulin excipients delivered with the insulin may be electrochemically cleaned from the electrode of the glucose monitor after each bolus dose to minimize or eliminate the effects of contaminant buildup on the electrode.

Additionally, or alternatively, the output signal causing delivery of the cleaning electrical current may be responsive to a user input to perform the electrochemical cleaning procedure. In this way, delivery of the cleaning electrical current to remove excipients from the electrode may be selectively chosen by the patient. In yet another example, the one or more processors may be configured to cause delivery of the cleaning electrical current in a regular sequence based on the passage of time. For example, the one or more processors may cause delivery of the cleaning electrical current when a first time period expires, and again when a second time period expires.

In some examples, the cleaning electrical current may be configured as one or more pulses that are in discreet steps (e.g., alternate between two or more different voltages such as a positive and negative voltage). Additionally, or alternatively, the cleaning electrical current may be configured to cyclically sweep a range of voltages.

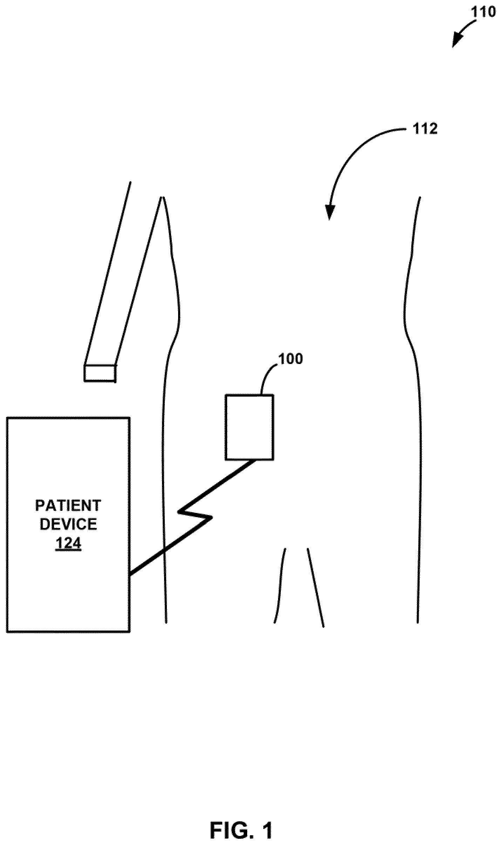

FIG. 1 is a block diagram illustrating an example glucose level management system in accordance with one or more examples described in this disclosure. FIG. 1 illustrates system 110 that includes patch pump 100. Patch pump 100 provides a housing that includes an insulin pump for delivery of insulin and a glucose level monitor comprising a glucose sensor, and patient device 124. As illustrated, patch pump 100 is connected to the stomach of patient 112. However, patch pump 100 may be connected to other locations as well, such as the arm, leg, etc. of patient 112.

As described in more detail, patch pump 100 includes a cannula connected to the insulin pump and implanted within patient 112 for delivery of insulin to patient 112, and the glucose sensor comprising a plurality of electrodes implanted within patient 112 used for sensing signal indicative of glucose level of patient 112. In some examples, patch pump 100 may be referred to as a "two-in-one" or "all-in-one (AIO) insulin pump".

In some examples, rather than a patch pump 100, the example techniques may be formed for a system in which the insulin pump and glucose level monitor are separate. In such examples, the insulin pump may be connected to an infusion set via tubing, where the infusion set attaches to patient 112 for delivery of insulin.

Patient 112 may be diabetic (e.g., Type 1 diabetic or Type 2 diabetic), and therefore, the glucose level in patient 112 may be controlled with delivery of supplemental insulin. For example, patient 112 may not produce sufficient insulin to control the glucose level or the amount of insulin that patient 112 produces may not be sufficient due to insulin resistance that patient 112 may have developed.

To receive the supplemental insulin, patient 112 may carry patch pump 100 along with patient device 112 that form system 100. The techniques described in this disclosure may be utilized with any insulin pump and/or glucose monitoring system that includes an in vivo glucose sensor (e.g., a continuous glucose monitor or other in vivo glucose sensor). The example techniques may be applicable to systems in which the glucose monitoring and the delivery of insulin are performed by different devices, such as in the MINIMED^{™} 770G insulin pump system.

Patch pump 100 may include a sensor that is inserted under the skin of patient 112 (e.g., in vivo), such as near the stomach of patient 112 or in the arm of patient 112 (e.g., subcutaneous connection). The sensor of patch pump 100 may be configured to measure the interstitial glucose level, which is the glucose found in the fluid between the cells of patient 112. Patch pump 100 may be configured to continuously or periodically sample the glucose level and rate of change of the glucose level over time. The sensor includes at least one sensor electrode.

In one or more examples, patch pump 100 and patient device 124 may together form a closed-loop therapy delivery system, which is often referred to as SAP therapy. For example, patient 112 may set a target glucose level (e.g., via patient device 124), usually measured in units of milligrams per deciliter. Patch pump 100 may receive the current glucose level and, in response, may increase or decrease the amount of insulin delivered to patient 112. For example, if the current glucose level is higher than the target glucose level, patch pump 100 may increase the insulin. If the current glucose level is lower than the target glucose level, patch pump 100 may temporarily cease delivery of the insulin. Patch pump 100 may be considered as an example of an automated insulin delivery (AID) device. Other examples of AID devices may be possible, and the techniques described in this disclosure may be applicable to other AID devices.

Patch pump 100 (e.g., with the insulin pump and the glucose monitor) may be configured to operate to mimic some of the ways in which a healthy pancreas works. Patch pump 100 may be configured to deliver basal dosages, which are small amounts of insulin released continuously throughout the day. There may be times when glucose levels increase, such as due to eating or some other activity that patient 112 undertakes. Patch pump 100 may be configured to deliver bolus dosages on demand in association with food intake or to correct an undesirably high glucose level in the bloodstream. In one or more examples, if the glucose level rises above a target level, then patch pump 100 may deliver a bolus dosage to address the increase in glucose level. Patch pump 100 may be configured to compute basal and bolus dosages and deliver the basal and bolus dosages accordingly, or the computation of the basal and bolus dosages may be offloaded to patient device 124. As an example, patch pump 100 may determine the amount of a basal dosage to deliver continuously and then determine the amount of a bolus dosage to deliver to reduce glucose level in response to an increase in glucose level due to eating or some other event.

Accordingly, in some examples, the glucose monitor of patch pump 100 may sample glucose levels for determining rate of change in glucose level over time. One or more processors of patch pump 100 may compare the glucose level to a target glucose level (e.g., as set by patient 112 or a clinician) and adjust the insulin dosage based on the comparison. It may be possible for patient device 124 to compare and adjust the insulin dosage alone or in combination with patch pump 100. In some examples, patch pump 100 may adjust insulin delivery based on a predicted glucose level (e.g., where glucose level is expected to be in the next 30 minutes).

As described above, patient 112 or a clinician may set one or more target glucose levels. There may be various ways in which patient 112 or the clinician may set a target glucose level. As one example, patient 112 or the clinician may utilize patient device 124 to communicate with patch pump 100. Examples of patient device 124 include mobile devices, such as smartphones, tablet computers, laptop computers, and the like. In some examples, patient device 124 may be a special programmer or controller (e.g., a dedicated remote control device) for patch pump 100. Although FIG. 1 illustrates one patient device 124, in some examples, there may be a plurality of patient devices. For instance, system 110 may include a mobile device and a dedicated wireless controller, each of which is an example of patient device 124. For ease of description only, the example techniques are described with respect to patient device 124 with the understanding that patient device 124 may be one or more patient devices.

In one or more examples, patient device 124 may comprise a user interface with which patient 112 or the clinician may control patch pump 100. For example, patient device 124 may comprise a touchscreen that allows patient 112 or the clinician to enter a target glucose level. Additionally or alternatively, patient device 124 may comprise a display device that outputs the current and/or past glucose level. In some examples, patient device 124 may output notifications to patient 112, such as notifications if the glucose level is too high or too low, as well as notifications regarding any action that patient 112 needs to take.

Due to the size of patch pump 100, the cannula to deliver the insulin and the glucose monitor may be relatively close to one another in vivo. The insulin that patch pump 100 delivers may not be only insulin. Rather, patch pump 100 delivers a fluid that includes insulin and one or more excipients (phenol, m-cresol, glycerin, etc.). While the excipients may be useful for ensuring that the insulin is properly delivered and absorbed, the excipients may impact the functionality of the glucose monitor.

For instance, the glucose monitor includes one or more electrodes (e.g., working electrode, counter electrode, and reference electrode, described in more detail). Patch pump 100 may determine a glucose level of patient 112 based on a signal current sensed by the one or more electrodes. However, the excipients in the delivered fluid may impact the impedance at the one or more electrodes (e.g., by coating the one or more electrodes, and changing the impedance). Insulin excipients from insulin doses (e.g., basal or bolus dosages) may cause the excipients to be oxidized and possibly lead to deposits and build up a thin film on the surface of the electrodes causing electrochemical interference and a change in the electrical parameters of the glucose monitor. The change in impedance may impact the sensed current level, resulting in potentially a less optimal glucose level measurement.

This disclosure describes example techniques to clean, in vivo, one or more electrodes of the glucose monitor of patch pump 100. For example, patch pump 100 may be configured to cause delivery of a cleaning electrical current to the sensor electrode to at least partially reverse the deleterious effects of deposits and/or thin films of excipients on the sensor, as will be further described below. In this way, the example techniques provide for a way in which to clean, in vivo, the electrodes resulting in less impact on the sensed current measurements. That is, the sensed current measurements better reflect the actual glucose level because the change in impedance at the electrodes of the glucose monitor is reduced.

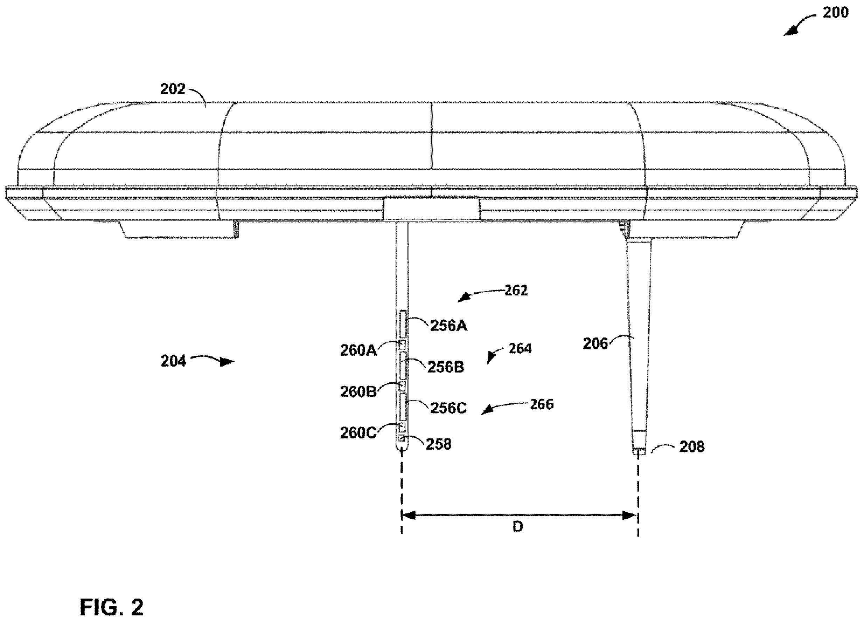

FIG. 2 is a cross-sectional view of an example medical device 200 including a glucose monitor and insulin pump. In the example shown, medical device 200 includes a glucose monitor and an insulin delivery device packaged together such that the electrode of the glucose monitor is placed relatively near an insulin delivery site on the patient. Medical device 200 is an example of patch pump 100 of FIG. 1.

Medical device 200 may inserted into patient 112 a single time and include both insulin delivery and glucose monitoring functionalities, e.g., so that patient 112 does not need to have two different devices inserted, e.g., two different insertions. In the example shown, medical device 200 includes housing 202, sensing portion 204 (e.g., which forms part of the glucose monitor), and delivery lumen 206 (e.g., which is an example cannula coupled to an infusion pump within medical device 200). Housing 202 may be configured to house the glucose monitor (not shown) and the insulin pump (not shown), such that sensing portion 204 and delivery lumen 206 may be inserted into patient 112 and separated laterally by a distance D.

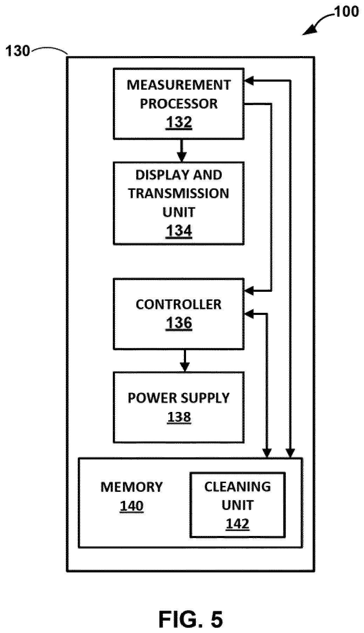

Sensing portion 204 may include glucose sensor electrodes 262, 264, and 266. In the example shown, sensing portion 204 includes three counter electrodes 256A, 256B, and 256C (collectively referred to as "counter electrodes 256"), and three working electrodes 260A, 260B, and 260C, (collectively referred to as "working electrodes 260"), which may form three glucose sensors, e.g., working-counter electrode pairs providing three separate sensor signals (iSigs). For example, counter electrode 256A and working electrode 260A may form an electrode pair that makes up glucose sensor electrode 262, counter electrode 256B and working electrode 260B may form an electrode pair that makes up glucose sensor electrode 264, and counter electrode 256C and working electrode 260C may form an electrode pair that makes up glucose sensor electrode 266. Sensing portion 204 also includes reference electrode 258. Each of counter electrodes 256, reference electrode 258, and working electrodes 260 may be substantially similar to counter electrode 356, reference electrode 358, and working electrodes 360 described below with reference to FIG. 5. Sensing portion 204 may be connected to the glucose monitor housed within housing 202.

Delivery lumen 206 may be connected to an infusion set and/or an insulin pump housed within housing 202, and may be configured to deliver a fluid that includes insulin and excipients via one or more apertures in fluid communication with a lumen of delivery lumen 206 and inserted within patient 112. In the example shown, delivery lumen 206 may output the fluid at distal end 208.

As described, the delivered fluid includes insulin and insulin excipient(s). The excipient concentration may be greatest nearest the insulin delivery site and may decrease as the excipients diffuse within the patient. In some examples, insulin excipients may be deposited on one or more of counter electrodes 256, reference electrode 258, and working electrodes 260, e.g., if D is small enough such that the excipients concentration is high enough near those electrodes, and cause electrochemical interference.

In some examples, housing 202 may be configured to have a lateral separation distance D between sensing portion 204 and insulin delivery lumen 206 of less than about 10 cm, or less than about 5 cm. In some examples, separation distance D between sensing portion 204 and insulin delivery lumen 206 may be less than about 2 centimeters. "About," as used herein, encompasses values within ten, twenty, or thirty percent of the stated value. In examples in which D is small enough such that insulin excipients may cause electrochemical interference, medical device 200 may be configured to deliver a cleaning electrical current to sensing portion 204 to electrochemically clean excipients from one or more of counter electrodes 256 an/or working electrodes 260.

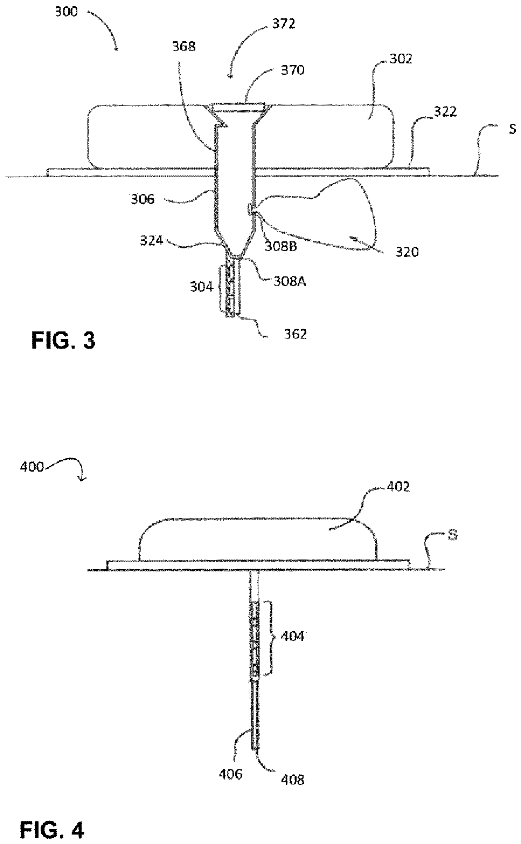

FIGS. 3 is a conceptual cross-section view of example medical device 300 according to the present disclosure. FIG. 4 is a conceptual side view of similar medical device 400. Medical device 300 of FIG. 3 and medical device 400 of FIG. 4 are configured to both provide glucose sensing and insulin delivery by inserting the sensing portion and insulin cannula together, with a single needle. Configured in this way, the examples of FIGS. 3 and 4 may reduce the overall footprint of the device, and/or the number of needle insertions may be reduced. These aspects may yield a reduction in foreign body response and/or can yield a reduction in foreign body response and reduce fewer sites with scarring in the hypodermis.

Device 300 of FIG. 3 includes housing 302. Channel 368 is formed through housing 302, such that sensing portion 304 and insulin cannula 306 may be inserted, together, with a single needle through septum 370 of port 372. In some examples, housing 302 may be attached to skin S of a patient (e.g., patient 112, FIG. 1) via adhesive layer 322, although other systems for mounting device 300 are considered. In the example of FIG. 3, insulin cannula 306 and sensing portion 204 extends from channel 368 into the body of the patient (e.g., a vascular or interstitial tissue volume within the patient). In some examples, insulin cannula 306 may be configured to be inserted into the patient after sensing portion 304, such that sensing portion 304 may be deeper (e.g., further under the patient's skin) than insulin cannula 306. Insulin cannula 306 may include one or more openings 308A, 308B which infusion media 320 (e.g., a fluid including glucose) may pass from device 300 into the patient. Sensing portion 304 may include sensor electrode 362.

Example device 400 of FIG. 4 may be generally described similarly to device 300 of FIG. 3, differing as described below. In some examples, as illustrated, sensing portion 404 may be configured to be inserted into the patient after insulin cannula 406, such that insulin cannula 406 may be deeper (e.g., further under the patient's skin) than sensing portion 404.

FIG. 5 is a block diagram illustrating components of a medical device in more detail. For instance, FIG. 5 is a block diagram of sensor electronics device 130 according to an example of the disclosure. Patch pump 100 (FIG. 1), medical device 200 (FIG. 2), medical device 300 (FIG. 3), and/or medical device 400 (FIG. 4) may include one or more processors configured to perform example techniques. Sensor electronics device 130 may be part of the one or more processors. In one or more examples, the glucose monitor may be considered as including the electrodes used for determining the glucose level. Sensor electronics device 130 may be configured to determine the glucose level, as well as cause delivery of electrical current to clean the electrodes, as described in this disclosure. Sensor electronics device 130 may be connected to a sensor electrode (262, 264, 266, FIG. 2). The sensor electrode may include a biomolecule or some other catalytic agent, and may be placed in a human body in a vascular or non-vascular environment. For example, the sensor electrode may be placed in a vein and be subjected to a blood stream, or may be placed in a subcutaneous or peritoneal region of the human body.

Sensor electronics device 130 may include measurement processor 132, display and transmission unit 134, controller 136, power supply 138, and memory 140.

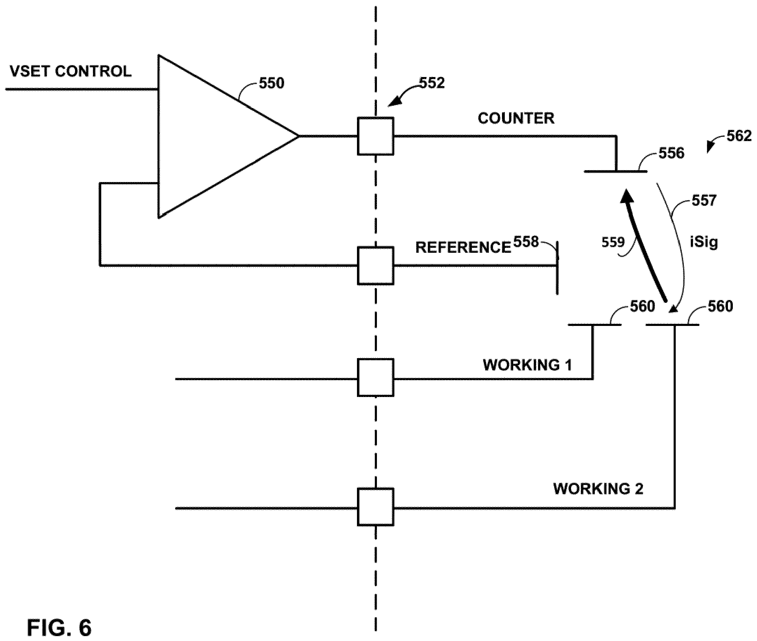

In examples of the disclosure, measurement processor 132, display and transmission unit 134, and controller 136 may be formed as separate semiconductor chips. However, other examples may combine measurement processor 132, display and transmission unit 134, and controller 136 into a single or multiple customized semiconductor chips. In general, measurement processor 132 may be configured to receive a current, voltage, and/or impedance from sensor electrodes such as electrodes 262, 264, and/or 266 (FIG. 2). The sensor electrodes may generate a sensor signal indicative of a concentration of a physiological characteristic being measured. For example, the sensor signal may be indicative of a glucose reading. The sensor signal may be measured at a working electrode 560 (FIG. 6) of the glucose sensor electrodes that from the glucose monitor. In an example of the disclosure, the sensor signal may be a current (e.g., iSig) measured at the working electrode 560 (FIG. 6). In another example of the disclosure, the sensor signal may be a voltage measured at the working electrode 560 (FIG. 6) of the sensor electrodes.

Measurement processor 132 receives the sensor signal (e.g., a measured current, voltage, and/or impedance) after the sensor signal is measured at the electrode (e.g., working electrode 560 (FIG. 6)). Measurement processor 132 may receive the sensor signal and calibrate the sensor signal utilizing reference values. For example, measurement processor 132 may calibrate the sensor signal utilizing reference values based on a known analyte quantity, e.g., a zero glucose measurement to determine a baseline sensor signal. In some examples, changes to the sensor over time may change the responsivity of the glucose sensor, changing the sensor signal and glucose measurement accuracy. Measurement processor 132 may utilize the reference values to adjust for changes over time. In an example of the disclosure, the reference values are stored in a reference memory (e.g., memory 140) and provided to measurement processor 132. Based on the sensor signals and the reference values, measurement processor 132 may determine a glucose measurement. Measurement processor 132 store the glucose measurements in memory 140. The sensor measurements may be sent to display and transmission unit 134 to be either displayed on a display in or transmitted to an external device.

Memory 140 may be any type of memory device and may be configured to store glucose measurements produced by measurement processor 132, reference values used to determine glucose measurements from sensor signals, or other data used and/or produced by measurement processor 132 and/or controller 136. In some examples, memory 140 may further store software and/or firmware that is executable by measurement processor 132 and/or controller 136. For example, memory 140 may store cleaning unit 142, which may store instructions that, when executed, causes delivery of a cleaning current to flow to one or more sensor electrodes, as will be further described below.

Sensor electronics device 130 may be a glucose monitor which includes a display to display physiological characteristics readings. However, such displays may not be needed, such as in examples where the display is provided by patient device 124.

Power supply 138 may be a battery. In other examples, different battery chemistries may be utilized, such as lithium-based chemistries, alkaline batteries, nickel metal hydride, or the like, and a different number of batteries may be used. Sensor electronics device 130 provides power to electrodes via power supply 138 through a cable and a cable connector. In some examples, power supply 138 may provide the energy for the cleaning electrical current.

Controller 136 may be a processor, digital signal processors (DSPs), application specific integrated circuits (ASICs), field programmable gate arrays (FPGAs), or any other equivalent integrated or discrete logic circuitry. In some examples controller 136 may be configured to cause a specific voltage or current to be output to glucose sensor electrodes 262, 264, 266 (FIG. 2). Glucose sensor electrodes 262, 264, 266 may receive the voltage level or value. With reference to FIGS. 5 and 6, in an example of the disclosure, a counter electrode 556 of glucose sensor electrodes may receive the reference voltage from power supply 138. The application of the voltage level VSET may cause glucose sensor electrode 562 to create a sensor signal (e.g., a signal current through working electrode 560) indicative of a concentration of a physiological characteristic being measured (e.g., blood glucose). Cleaning unit 142 may cause controller 136 to cause glucose sensor electrode 662 to switch from the sensing mode described above to a cleaning mode by causing delivery of a cleaning electrical current 559 to glucose sensor electrode 562. In some examples, the cleaning electrical current may be a current which, similar to signal current 557, travels from counter electrode 556 to working electrode 560. In some examples, the cleaning electrical current may be amplified in voltage level or current flow relative to iSig 557. Additionally, or alternatively, controller 136 may cause cleaning electrical current 559 to travel in a direction opposite iSig, from working electrode 560 to counter electrode 556. In another example, cleaning electrical current 559 may toggle between being sourced at counter electrode 556 and working electrode 560.

FIG. 6 is a block diagram illustrating example glucose sensor electrode 562 and a bias voltage being applied to glucose sensor electrode 562 according to an example of the disclosure. In the examples shown, example sensor electrodes include counter electrode 556, reference electrode 558, and working electrodes 560, which may be examples of glucose sensor electrodes that form a glucose monitor and may be used in a glucose and oxygen sensor having a glucose oxidase (GOx) enzyme catalyzing a reaction with electrodes 556, 558, 560. In the example in FIG. 6, an operational amplifier (op amp) 550 or other servocontrolled device may connect to the glucose sensor electrodes through a circuit/electrode interface 552. Op amp 550, utilizing feedback through the glucose sensor electrodes, maintains a prescribed voltage between reference electrode 558 and a working electrode 560 (e.g., VSET) by adjusting the voltage at counter electrode 556. In some examples, the voltage at reference electrode 558 is from about -1000 mV to about 1000 mv. In some examples, a positive voltage may cause a current to be source at counter electrode 556 and sunk at working electrode 560, while a negative voltage may cause a current to be sourced at working electrode 560 and sunk at counter electrode 556.

In normal sensing mode operation, current 557 (iSig) may flow from a counter electrode 556 to a working electrode 560. Counter electrode 556 balances the chemical reaction that is occurring at working electrode 560. Measurement processor 132 of FIG. 5 may measure current 557 to determine the electrochemical reaction between the glucose sensor electrodes. The circuitry disclosed in FIG. 6 may be utilized in a long-term or implantable sensor or may be utilized in a short-term or subcutaneous sensor.

In this way, sensor electronics device 130 may monitor an electrical parameter of the electrical chemical cell, e.g., an electrical current that is proportional to the impedance of the electrochemical cell such as iSig 557. For example, measurement processor 132 may provide a sensor signal to controller device 136. In one example, the sensor signal is a current (iSig 557) through a working electrode 560 of sensor electrode 562. Controller 136 may accumulate current values over a period of time and may calculate a rate of change of the current values. For example, controller 136 may calculate the slope of a plot of the current (e.g., iSig 557).

In some examples, controller 136 may accumulate and measure changes in electrochemical impedance spectroscopy (EIS) (real and imaginary impedance at different frequencies), voltage at a counter electrode 556, and/or current through a background electrode. A background electrode is similar to a working electrode 560, but may not include a Gox layer for breaking down and sensing glucose. Like current through a working electrode 560, larger slopes/changes in these other sensor signals may indicate a need for a higher VSET, whole lower slopes/changes in these other sensor signals may indicate a need for a lower VSET at the working electrode 560. In this way, sensor electronics device 130 may be configured to monitor EIS, voltage, and/or current through a background electrode similar to monitoring iSig 557 as described herein.

In some examples, sensor electronics device 130 may be configured to monitor iSig 557 or other electrical parameters and determine when a monitored parameter (e.g., iSig 557) satisfies a threshold. In some examples, sensor electronics device 130 may be configured to determine whether iSig 557 satisfies a threshold rate of change. In some examples, sensor electronics device 130 may be configured to determine when the impedance at glucose sensor electrode 562 may satisfy a threshold or satisfy a threshold rate of change.

For example, a spike in iSig 557 may indicate a rise in glucose, or a spike in iSig 557 may indicate a rise in interferents such as insulin excipients. The threshold value may be predetermined, at least initially, and stored in memory 140. The threshold value may be adjusted over time, e.g., so as to compensate for changes to the causes of electrochemical interference over time such as changes to a thin film of insulin excipients deposited on an electrode of the electrochemical cell, e.g., any of electrodes 262, 264, 266 (FIG. 2). The threshold value may be predetermined, adjusted, or determined based on previous iSig 557 measurements, e.g., for comparison to determine if there is a change to an average value of an iSig 557 spike, or based on any other information indicating an iSig 557 value at which a diagnostic test should be performed to determine whether electrochemical interference is present and/or has changed. In other words, sensor electronics device 130 may monitor iSig 557.

Returning to FIG. 5, as discussed above, due to electrochemical interference from deposition of insulin excipients, patch pump 100 or medical device 200 may provide inaccurate readings to patient 112. As such, cleaning unit 142 may be executed to repair the poisoned working electrode 560 (FIG. 6). Either of measurement processor 132 and/or controller 136 may be configured to execute cleaning unit 142. Although cleaning unit 142 is illustrated as software that executes on measurement processor 132 and/or controller 136, in some examples, cleaning unit 142 may be fixed-function circuitry that is formed within measurement processor 132 and/or controller 136 or independent of measurement processor 132 and/or controller 136. In some examples, cleaning unit 142 may be firmware that measurement processor 132 and/or controller 136 execute.

With concurrent reference to FIGS. 5 and 6, in some examples, responsive to measurement processor 132 determining that iSig 557, an impedance (real, imaginary, or both), or another electrical parameter satisfies a threshold or a threshold rate of change, sensor electronics device 130 may be configured to interrupt the monitoring of iSig 557 to deliver cleaning electrical current 559 via instructions stored in cleaning unit 142. For example, as illustrated, cleaning unit 142 may be configured to change or adjust the voltage at counter electrode 556 to change counter electrode 556 to a current sink such that cleaning current 559 may flow in a direction opposite iSig 557. Additionally, or alternatively, the voltage at counter electrode 556 may be amplified (e.g., increased) such that additional current flows from counter electrode 556 to working electrode 560 during the one or more cleaning pulses. Cleaning unit 142 may employ a single pulse or multiple pulses of cleaning electrical current in either direction and at varying amplitude, defining a waveform, as will be further described below. Accordingly in some examples, cleaning electrical current 559 may be sourced from working electrode 560 and sunk at counter electrode 556, or cleaning current may be sourced from counter electrode 556 and sunk at working electrode 556, or may be toggled between being sourced from counter electrode 556 and sunk at working electrode 560, and sourced from working electrode 560 and sunk at counter electrode 556.

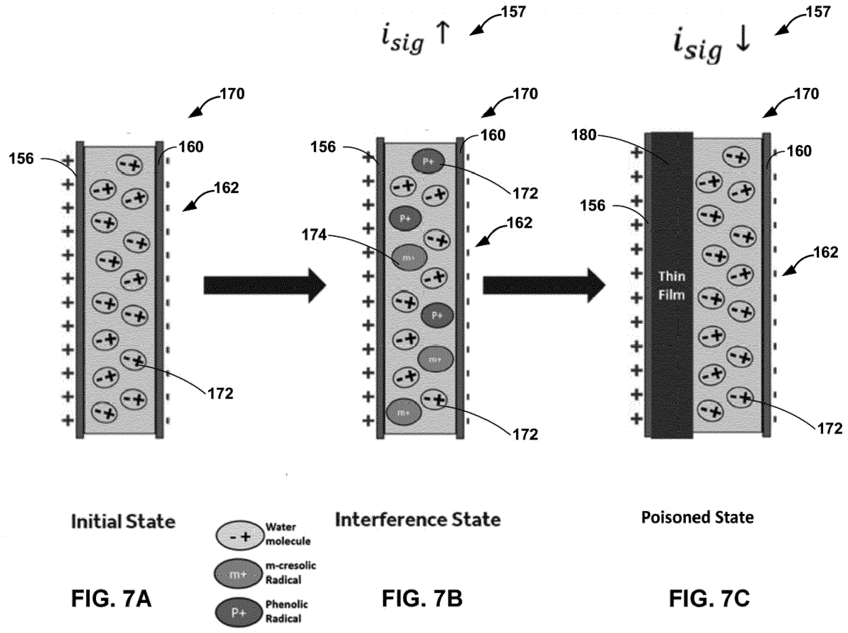

FIGS. 7A - 7C illustrate the buildup of a thin film on sensor electrode 162 of an electrochemical cell due to insulin excipients, and are described concurrently below. FIG. 7A is a cross-sectional view of a portion of an example electrochemical cell in an initial state, FIG. 7B is a cross-sectional view of a portion of an example electrochemical cell in an interference state, FIG. 7C is a cross-sectional view of a portion of an example electrochemical cell in a poisoned state. The illustrated electrochemical cell may be a portion of patch pump 100 of FIG. 1, device 200 of FIG. 2, device 300 of FIG. 3, or device 400 of FIG. 4.

In the example shown, the electrochemical cell of the glucose monitor is in an initial state. In operation without electrochemical interference, the electrochemical cell outputs iSig 157 in response to the presence and/or amount of an analyte within interstitial fluid 170, e.g., glucose. In the example shown, interstitial fluid 170 comprises mostly water, e.g., water molecules 172. In the example shown, iSig 157 (not shown in FIG. 7A) or changes to iSig 157 may be proportional to an amount of glucose present or changes to the amount of glucose present in interstitial fluid 170.

When a bolus of insulin is provided to the patient, e.g., via patch pump 100 (FIG. 1), medical device 200 (FIG. 2), medical device 300 (FIG. 3), or medical device 400 (FIG. 4), interstitial fluid 170 may contain insulin and insulin excipients such as phenol, glycerin, m-cresol, or the like. For ease of description, phenol and m-cresol will be described herein, but other excipients are also considered. When these interfering compounds come in contact with sensor electrodes, the bias voltage may cause them to oxidize to phenolic radicals 172 and m-cresolic radicals 174, as shown in FIG. 7B. As illustrated, the presence of phenolic radicals 172 and m-cresolic radicals 174 causes additional charge transfer to transfer to the sensor electrodes, which may cause iSig 157 to increase artificially (e.g., not in response to an increase in glucose). In some examples, as insulin causes patient 112 to absorb the glucose, the glucose concentration within interstitial fluid 170 decreases, causing iSig 157 to decrease. However, the insulin excipients cause iSig 157 to increase (e.g., the electrochemical interference with the measurement of the analyte of interest).

After a time, the phenolic radicals 172 and m-cresolic radicals 174 may electropolymerize and deposit on counter electrode 156 as illustrated in FIG. 7C, and/or on working electrode 160, and build up a thin film 180. Thin film 180 may change the electrical responsivity of the electrochemical cell. As illustrated in FIG. 7C, thin film 180 may change the responsivity of the electrochemical cell to an analyte within solution 170. For the same concentration of an analyte, such as glucose, within solution 170, the signal response iSig 157 of the electrochemical cell including thin film 180 as illustrated in FIG. 7C will be less than the signal response iSig 157 of the electrochemical cell without thin film 180 as illustrated in FIG. 7A. In other words, the insulin excipients can cause both short term effects due to oxidation, and long term effects due to depositing as a thin film 180, which can change the long-term or permanent electrical configuration of the electrochemical cell, e.g., the insulin excipients cause long-term electrochemical interference illustrated in FIG. 7C, and insulin excipients in solution 170 cause short-term electrochemical interference as illustrated in FIG. 7B, e.g., while the insulin excipients remain in solution.

In some examples, removal of thin film 180 may be accomplished by removal of the medical device and physically scrubbing or polishing sensor electrode to return it to the initial condition of FIG. 7A. However, such removal may be burdensome and may be done improperly without proper sanitization. In one or more examples, rather than removing the device to clean counter electrode 156, working electrode 160, or both, a cleaning electrical current delivered to electrode 162 may at least partially remove thin film 180 of FIG. 7C, and may return sensor electrode 162 to a state the same or similar to the initial state illustrated in FIG 7A.

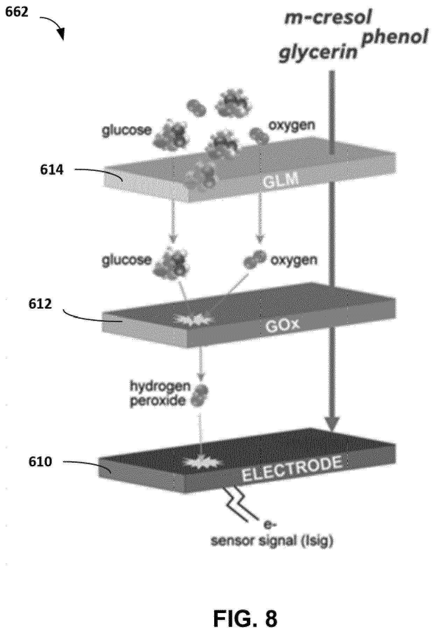

FIG. 8 is a schematic diagram illustrating the buildup of a thin film on sensor electrode 662 of an electrochemical cell due to insulin excipients m-cresol, phenol, and glycerin. Sensor electrode 662 may be an example of a portion of sensor electrodes 262, 264, or 266 of FIG. 2, or sensor electrode 162 of FIGS. 7A-7C. As such, sensor electrode 662 may be used in a glucose and oxygen sensor having a glucose oxidase (GOx) enzyme catalyzing a reaction with glucose sensor electrode 662. The Gox enzyme may be formed as Gox layer 612. Gox layer 612 may be disposed on electrode substrate 610, with or without one or more intervening layers. Electrode substrate 610 may include platinum or another metal or alloy. Positioned over GOx layer 612, with or without one or more intervening layers, may be glucose limiting membrane (GLM) 614. GLM 614 may also be called an interference rejection membrane. GLM 414 may be configured to regulate the influx of glucose to electrode substrate 610, which is the sensing layer of glucose sensor electrode 662, and may also reduce the influx of interfering agents such as insulin excipients. However, in some examples, as illustrated, at least a portion of the insulin excipients such as m-cresol, phenol, and/or glycerin may reach electrode substrate 610 and block glucose sensor electrode 662 from properly sensing glucose in the surrounding tissue. As such, in some examples, delivery of a cleaning electrical current as described herein may be desirable rather than or in addition to inclusion of GLM 614 on electrode 662.

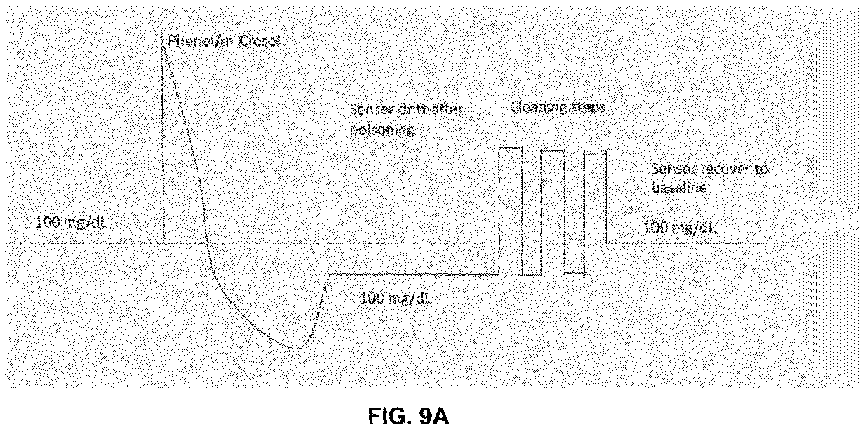

FIG. 9A is a conceptual chart illustrating an example cleaning pulse on an example poisoned electrode. The chart illustrates the sensed glucose concentration by, for example, sensor electrode 262 (FIG. 2). Starting from the left, during a first time segment, the sensor may sense a relatively constant glucose concentration, for example 100 mg/dL. Then, a bolus of insulin may be delivered near the sensor, which may cause an artificial spike in the sensed glucose concentration, illustrated at the phenol/m-Cresol spike of FIG. 9A and described above with respect to FIG. 7B. Then, as described above with respect to FIG. 7C, the sensor may have a film formed due to polymerization and deposit of insulin excipients. The sensor, now poisoned by formation of the thin film, may "drift," now sensing a reduced concentration of glucose in a tissue or blood sample that actually has the same or a similar level of glucose as the sample had before the drift occurred due to the formation of the thin film. One or more electrochemical cleaning pulses, illustrated as "cleaning steps" in FIG. 9A, may at least partially remove the thin film, reducing or eliminating the sensor drift and causing the sensor to measure and reflect the correct concentration of glucose in the blood or tissue sample. In this way, the electrode poisoning effect due to delivery of fluid including insulin and excipients near the sensor may be addressed by cleaning the sensor in vivo.

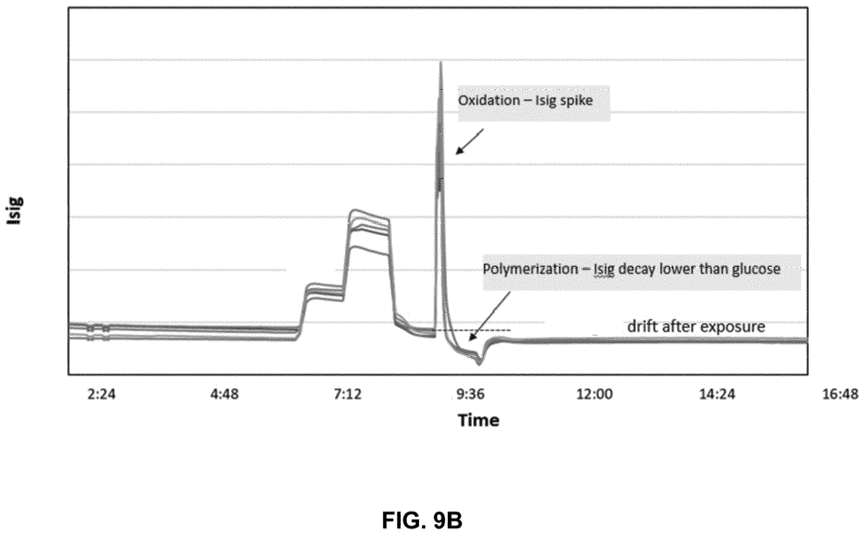

FIG. 9B is a chart illustrating an example interference testing conducted on an example sensor electrode. The short term and long term effects of insulin excipients, which were m-cresol and phenol in the illustrated example, are demonstrated. In the test, which was performed in-vitro in a beaker, a 100 mg/dL glucose solution was monitored by a sensor electrode. Initially, the solution was measured by the sensor, which applied a VSET voltage of 535 mV. The measured signal current may be converted to a glucose level, as described above with respect to FIG. 1.

Next, a mixture of 1.5 mg/ml phenol and 1.72 mg/ml of cresol was added to the 100 mg/dL glucose solution. Introduction of the excipients (mimicking a bolus dosage of insulin) may have caused a short-term spike in the signal current iSig, as described with respect to the interference state of FG. 7B above. Following the initial spike, the signal current decayed to measure less than the actual concentration of glucose in the solution, which may correspond to the poisoned state described with respect to FIG. 7C above.

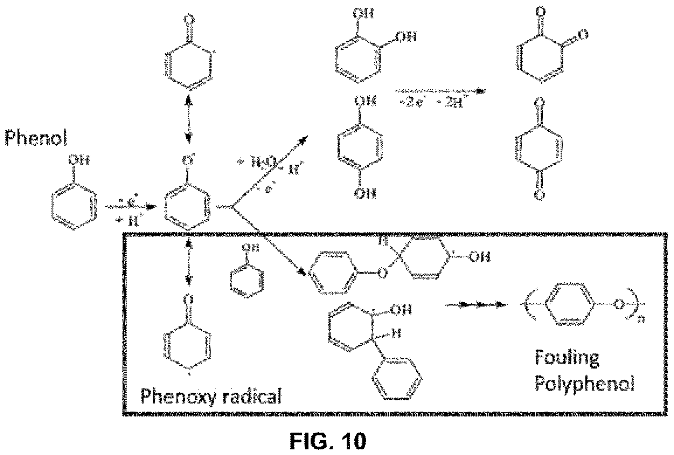

FIG. 10 is a schematic diagram illustrating an example polymerization pathway of phenol, an example insulin excipient. The one-electron oxidation of phenol may create a phenoxy radical, as described with FIG. 7B above. The phenoxy radical may react with other phenoxy radicals to create a fouling polyphenol polymer. The polyphenol polymer may deposit and build up on working electrode 160 as thin film 180 (FIG. 7C).

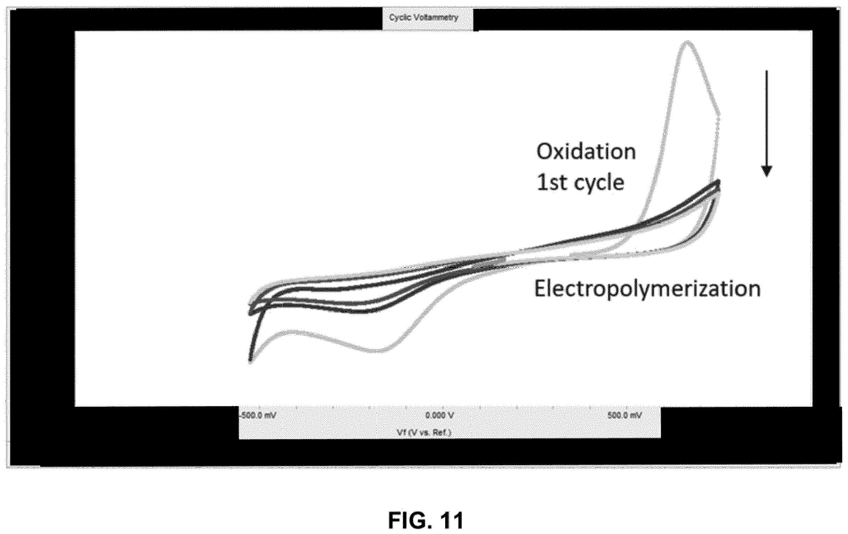

FIG. 11 is a chart illustrating an example electrochemical pulse using a cyclic voltammetry technique. The chart illustrating phenol interference of an example electrode, such as sensor electrodes 262, 264, or 266 of FIG. 2, or sensor electrode 162 of FIGS. 7A-7C. The poisoning effect was confirmed with cyclic voltammetry (CV). Phenol oxidation may occur in a range of from about 400mV to about 600 mV. As discussed above, the signal current (iSig) may be in or near this range, which may make both sensing and delivering glucose in the same device difficult, because the signal current may at least partially drive the excipients delivered with the insulin to oxidize, polymerize, and/or deposit on the sensor electrode. In some examples, as illustrated the VSET level (FIG. 6) may be configured to sweep through a range of voltages (e.g., from about -500 mv to about 3000 mV, such as from about -500 mV to 1000 mV). In some examples, the VSET level may be scanned through a range of voltages at a scan rate. In some examples, the cleaning electrical current (y-axis) may be delivered according to the varied VSET voltage. The range of voltages may be scanned through one or more times, which may define the pulse or pulses of cleaning electrical current.

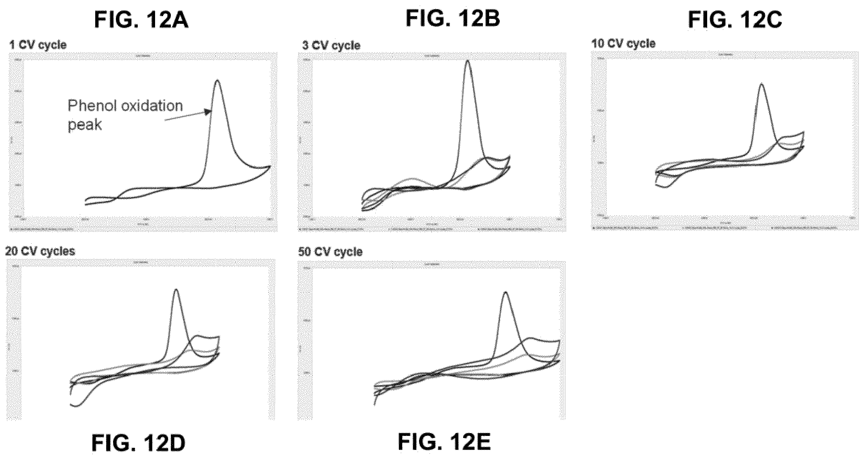

FIGS. 12A-12E are charts illustrating a cleaning electrical current delivered via cyclic voltammetry after 1, 3, 10, 20, and 50 cyclic voltammetry cycles, respectively. To generate the illustrate data, a polyphenol/poly m-cresol film ware form on a platinum electrode. Multiple cyclic voltammetry scans were performed on the platinum electrode, and the voltage was varied between -500 mV and 1000 mV in each cyle, and the buffer solution was phosphate-buffered saline (PBS). The scan rate in the illustrated example was 30 mV/second. The surface of the electrode was tested using chronoamperometry in hydrogen peroxide. A poisoned electrode will have no or low signal response to hydrogen peroxide, while a clean electrode will have a relatively high signal response compared to a poisoned electrode As illustrated by the increased current with progressive cycles through the range of voltages, an electrochemical cleaning pulses or pulses delivered by varying the VSET potential may clean a deposited thin film from an electrode. In some examples the oxidation potential may shift.

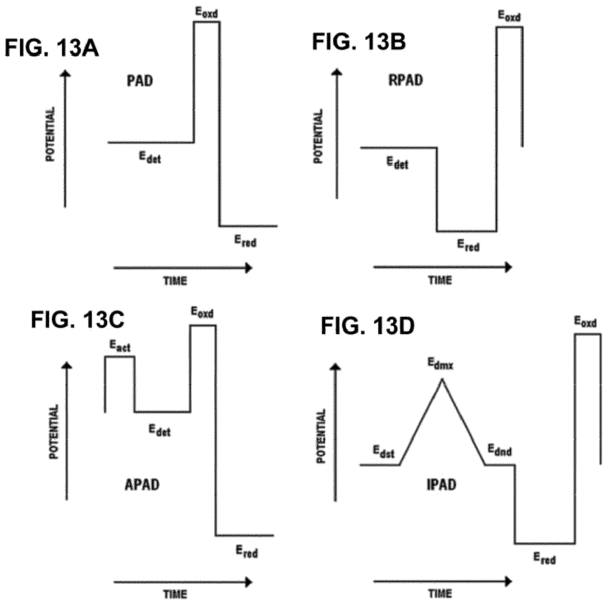

As mentioned above, cleaning unit 142 (FIG. 3) may cause a cleaning electrical current to be delivered as a single pulse or multiple pulses of cleaning electrical current in either direction and at varying amplitude, defining a waveform. FIGS. 13A-13D are schematic illustrations of example waveforms of an electrochemical cleaning pulse or pulses. For example, the cleaning current may be delivered as a series of potential steps or staircases to remove deposits associated with delivery of insulin on an electrode. In some examples, the electrode may be a microelectrode, where cleaning by a polishing method may be difficult or impossible. The waveform may be configured to achieve a more complete removal of excipients. For example, the polarity of the working electrode may be switched between high and low or positive and negative, as described above with respect to FIG. 6. A cleaning electrical current 559 (FIG. 6) may be delivered as a pulse or pulses. The pulse or pulses may be repeated for one or more cycles, for example until the desired sensor response is achieved (e.g., a measured signal current matches or is within a threshold matching percentage of an expected signal).

With concurrent reference to FIG. 6 and FIGS. 13A-13D, in some examples the waveform defined by plotting the potential between counter electrode 556 and working electrode 560 may be a three-step waveform. The three-step waveform may be defined as a single pulse of a plurality of pulses, where the illustrated waveforms may be repeated. In a three-step waveform, the electrode potential may first be stepped to the optimum value for detection of the electroactive species by the one or more processors (Edet, which may correspond to a VSET level for monitoring glucose). Following a delay time (tdel), during which the current mainly from double-layer charging is permitted to decay to minimal value, the analytical signal may be computed by integrating the faradaic current due to analyte oxidation for a specified time period (tint). The potential may then be stepped to a more positive value (Eoxd) at which the electrode surface is oxidatively cleaned of any compounds adsorbed during the detection step.

The potential may then stepped to a negative value (Ered) to reduce the oxide layer formed at Eoxd, thereby regenerating the electrode. As such, in some examples, the electrochemical cleaning pulse may define a segmented waveform which may include a first segment in which the one or more processors are configured to detect electroactive species, a second segment configured to oxidize an analyte associated with the delivery of insulin through the infusion set, and a third segment configured to reduce an oxide layer formed during the oxide step to at least partially regenerate the glucose monitor. In some examples, the first segment of the segmented waveform defines a voltage amplitude of from about 100 millivolts to about 1500 millivolts, the second segment defines a voltage amplitude of from about -1000 millivolts to about 2000 millivolts, and the third segment defines a voltage amplitude of from about -2000 to about 1000 millivolts.

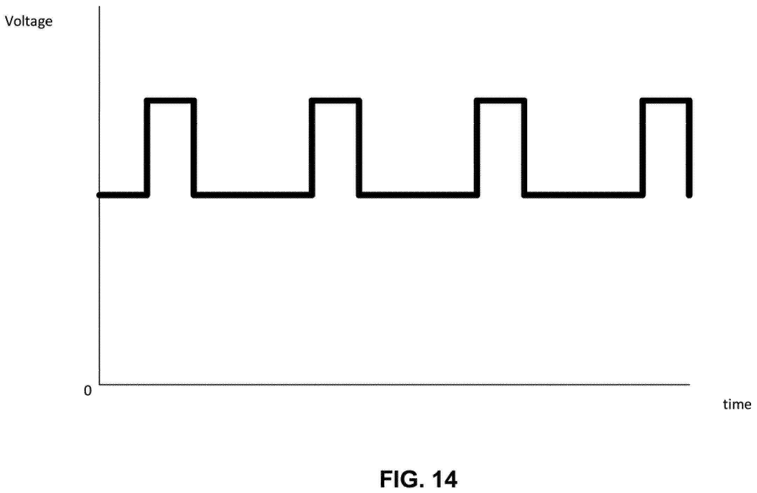

FIG. 14 is a schematic illustration of another example waveform of an electrochemical cleaning pulse. The illustrated pulsing steps were measured with a Gamry-Stingray potentiostat. The illustrated waveform includes a two-step waveform, and includes a plurality of pulses at varying potential and time. The electrochemical cleaning process may start at the potential for monitoring, which may be between about 100 mV and about 1500 mV, such as 850mV. The electrochemical cleaning process may commence by varying the voltage between 535 mV and 1070mV, and holding at each step for a period of from about 30 seconds to about 5 minutes. In , the series of pulses included a first segment at 535 mV for a duration of 120 seconds, a second segment at 1070 mV for a duration of 90 seconds, a third segment at 535 mV for a duration of 120 seconds, a fourth segment at 1070 mV for a duration of 30 seconds, a fifth segment at 535 mV for a duration of 120 seconds, a sixth segment at 1070 mV for a duration of 30 seconds, and a seventh segment at 535mV for a duration of 120 seconds.

In some examples, devices according to the present disclosure may be configured to deliver an initialization current to a sensor electrode as part of an initialization sequence upon placement of the device in vivo. In some examples, the initialization sequence may match at least a portion of the one or more pulses delivered as the cleaning electrical current. For example, the initialization sequence may include a plurality of pulses, each pulse of the plurality of pulses defining a voltage, a current, and/or a duration. The one or more processors may be configured to cause delivery of the cleaning electrical current as an electrochemical cleaning pulse or set of pulses that is substantially equal to at least one of the plurality of pulses in the initialization sequence in voltage, current, or duration. Substantially equal, as defined herein, includes equal voltages, currents, and durations as well as those voltages, currents, and durations within ten percent, within twenty percent, or within thirty percent of the reference voltage, current or duration.

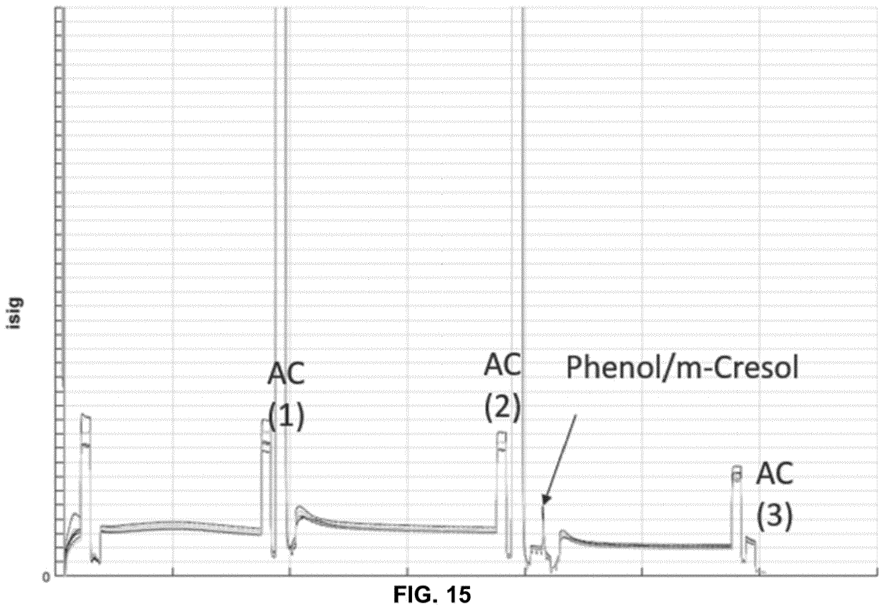

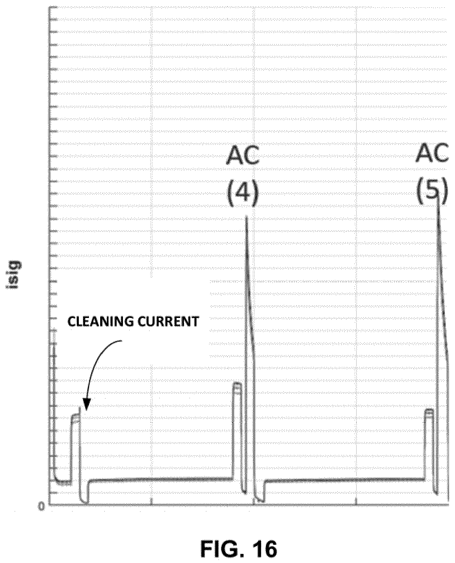

FIGS. 15 and 16 are charts illustrating the observed response of an example sensor before and after an electrochemical cleaning pulse as disclosed herein. The same sensors were used in the first round of testing illustrated in FIG. 15 and the second round of testing illustrated in FIG. 16. The sensor was exposed to a sample of acetaminophen at five different times, labeled AC (1) through AC (5) in FIGS. 12 and 13. Although the sensed analyte was acetaminophen in the illustrated example of FIGS. 15 and 16, it should be understood that the illustrated electrode poisoning and cleaning mechanism would occur where the sensed analyte is glucose, such as in glucose monitors according to the present disclosure. Thus, the illustrated testing is meant merely to convey that an electrode may be poisoned by phenol and m-cresol, then electrochemically cleaned by delivery of an electrochemical cleaning current, regardless of what substance acts as the sensed analyte.

The first two exposures to acetaminophen generated a spike in the signal current, as expected, due to the increased analyte concentration. After the second exposure to acetaminophen, the sensor electrode was exposed to a mixture of phenol and m-cresol similar to as described above with respect to FIG. 9, at the point called out in FIG. 15. The third exposure, labeled AC (3) in FIG. 15, generated a much smaller spike in the signal current relative to AC (1) and AC (2), which may be a result of the sensor electrode being in a poisoned state as described in FIG. 7C. Turning to FIG. 16, which extends the time period displayed in FIG. 15, the example electrochemical cleaning procedure of FIG. 14 above was performed on the sensor electrode between AC (3) and AC (4). After the electrochemical cleaning procedure, the fourth and fifth exposures to acetaminophen, labeled as AC (4) and AC (5) generated similar spikes as the first and second exposures. This test may demonstrate that performing an electrochemical cleaning procedure as described herein may at least partially remove excipient buildup on a sensor electrode.

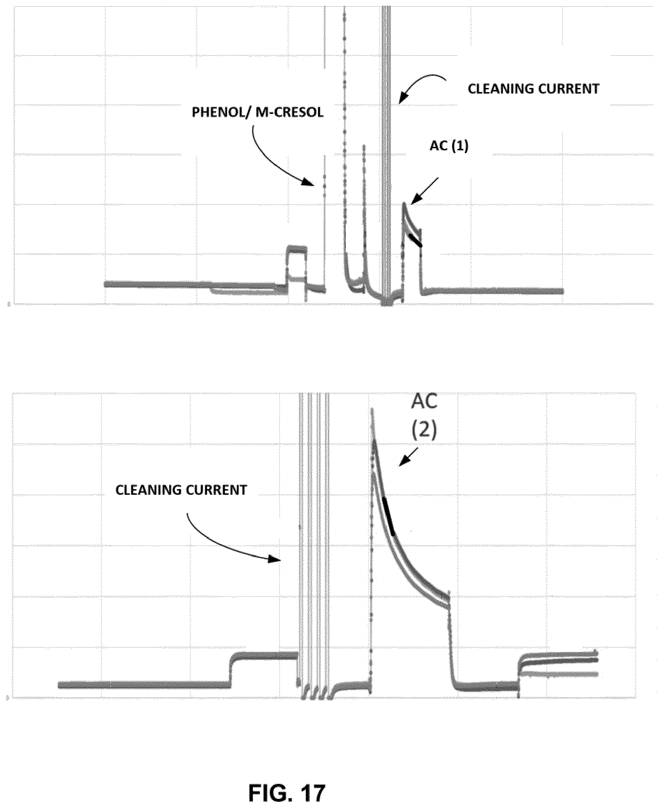

FIG. 17 illustrates the observed response of an example sensor before and after an electrochemical cleaning pulse. Similar to FIGS. 14 and 15, a sensor was exposed to a solution of acetaminophen as the sensed analyte and the signal current was monitored. Although acetaminophen solution was used as the sensed analyte in the illustrated example, glucose or another sensed analyte may be used in other examples. In this instance, the electrode was poisoned by exposure to a phenol/m-cresol solution before the first exposure, labeled AC (1) in the top chart. Then an electrochemical cleaning procedure was conducted, as described with respect to FIG. 14. The cleaning pulses are labeled as "cleaning current" in the top picture. After the electrochemical cleaning procedure, the sensor electrode was exposed to acetaminophen and the spike and plateau in the signal current were monitored. The following day, without an additional exposure to the phenol/m-cresol mixture, the electrochemical cleaning procedure was repeated, and the spike and plateau in the signal current was again monitored, illustrated as AC (2) in the bottom picture. After the second electrochemical cleaning procedure, the observed response was a higher spike followed by a plateau at closer to the expected value. The first electrochemical cleaning procedure (top) may have removed some excipient buildup from the electrode, and the second electrochemical cleaning procedure may have further removed additional excipient. This testing may indicate that the electrochemical cleaning procedure may be completed more than once to return the sensor electrode to an initial state after exposure of excipients (e.g., a bolus dosage of insulin.).

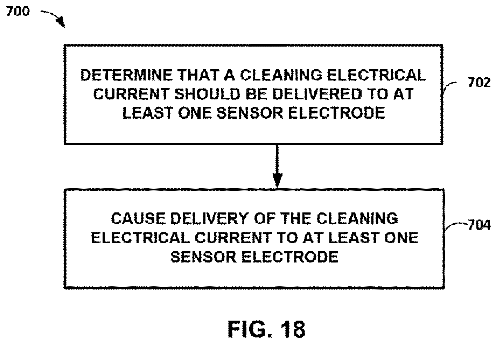

FIG. 18 is a flowchart illustrating example technique 700 according to the present disclosure. The technique of FIG. 85 is described with respect to sensor electronics device 130 of patch pump 100 of FIG. 1, medical device 200 of FIG. 2, medical device 300 of FIG.3, and medical device 400 of FIG. 4, but technique 700 may be performed with other devices, the described medical device may be used to perform other techniques. Patch pump 100 of FIG. 1, medical device 200 of FIG. 2, medical device 300 of FIG. 3, or medical device 400 of FIG. 4 may include sensor electronics device 130 of FIG. 3 as all or a portion of the processing circuitry of the device.

With concurrent reference to FIGS. 5, 6, and 18, technique 700 includes determining, by sensor electronics device 130 that a cleaning electrical current 559 should be delivered to at least one sensor electrode 562 (702). In some examples, determining that cleaning electrical current 559 should be delivered may include determining an impedance at sensor electrode 562 or signal current iSig 557 sensor electrode 562. In some examples, sensor electronics device 130 may determine that an impedance at sensor electrode 562 satisfies an impedance threshold or a threshold rate of impedance change to determine that cleaning electrical current 559 should be delivered to sensor electrode 562. Additionally, or alternatively, a threshold may be satisfied by iSig 557 or the rate of change of iSig 557 may satisfy a threshold or a threshold rate of change.

In some examples, determining that cleaning current 559 should be delivered to sensor electrode 562 may include receiving, by sensor electronics device 562, a representation of user input. For example, patient 112 (FIG. 1) or a clinician may input instructions to patient device 124 to perform an electrochemical cleaning procedure. Alternatively, in some examples determining that cleaning electrical current 559 should be delivered to sensor electrode 562 may include determining, by sensor electronics device 130, that a time period has expired.

In some examples, sensor electrode 562 may sense a change in impedance or iSig 557, and sensor electronics device 130 may determine that the change is due to delivery of a bolus dosage of insulin. Step 702 may include determining that cleaning electrical current 559 should be delivered based on a determination that a bolus dosage of insulin has been delivered. In some examples, step 702 may include delaying the determination that cleaning electrical current 559 should be delivered by one minute, 2 minutes, 5 minutes, or the like after determining that a bolus dosage of insulin has been delivered.

Technique 700 also includes causing delivery of the cleaning electrical current to at least one sensor electrode (704). In some examples, sensor electronics device 130 may cause cleaning current 559 to be delivered from a source at working electrode 560 to a sink at counter electrode 556. Additionally, or alternatively, cleaning current 559 may be sourced at counter electrode 556 and sunk at working electrode 560. In some examples, sensor electronics device 130 may toggle cleaning electrical current between being sourced from counter electrode 556 and sunk at working electrode 560, and sourced from working electrode 560 and sunk at counter electrode 556.

In some examples, causing delivery of cleaning current 559 may include varying, by sensor electronics device 130, the electrical potential at counter electrode 556 or working electrode 560. The varying potential may define a waveform relative to time. The waveform may be segmented, as illustrated in FIGS. 13A-13D and 14. In some examples, a first segment of the segmented waveform defines a voltage amplitude of from about 500 millivolts to about 1500 millivolts. In some examples, the waveform may include a three-step waveform, or a two-step waveform, or another number of steps or segments. In examples where the waveform is segmented into three steps, the segmented waveform may include a first segment wherein sensor electronics device 130 detects electroactive species (Edet, FIGS. 13A-13D). The segmented waveform may further include a second segment configured to oxidize an analyte associated with the delivery of insulin through the infusion set (Eoxd, FIGS. 13A-13D). The segmented waveform may further include a third segment configured to reduce an oxide layer formed during the oxide step to at least partially regenerate the glucose monitor (Ered, FIGS. 13A-13D).

In some examples, the technique of FIG. 18 may further include performing an initialization sequence upon placing sensor electrode 562 in vivo. In some examples, the initialization sequence includes a plurality of pulses, and each pulse of the plurality of pulses defines a voltage, current, and a duration. In some examples, at least one of the one or more electrochemical cleaning pulses are substantially equal to at least one of the plurality of pulses in the initialization sequence in voltage, current or duration.

In some examples, cleaning electrical current 559 may be a first cleaning electrical current, and the technique of FIG. 18 may include causing delivery of a second cleaning electrical current. In some examples, sensor electronics device 130 may determine that first cleaning electrical current 559 did not sufficiently regenerate a poisoned electrode (e.g., electrode 162 of FIG. 7C). For example, sensor electronics device 130 may compare a signal current (iSig 557, FIG. 6) to an expected signal current stored in memory 140 or calculated. Sensor electronics device 130 may determine that first cleaning electrical current 557 did not return the signal current to match or fall within a threshold matching percentage of an expected signal current. Responsive to this determination, sensor electronics device 130 may cause delivery of a second cleaning electrical current.

The techniques described in this disclosure may be implemented, at least in part, in hardware, software, firmware or any combination thereof. For example, various aspects of the described techniques may be implemented within one or more processors, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit comprising hardware may also perform one or more of the techniques of this disclosure.

Such hardware, software, and firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

The techniques described in this disclosure may also be embodied or encoded in a computer-readable medium, such as a computer-readable storage medium, containing instructions. Instructions embedded or encoded in a computer-readable medium may cause a programmable processor, or other processor, to perform the method, e.g., when the instructions are executed. Computer-readable media may include non-transitory computer-readable storage media and transient communication media. Computer readable storage media, which is tangible and non-transitory, may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer-readable storage media. It should be understood that the term "computer-readable storage media" refers to physical storage media, and not signals, carrier waves, or other transient media.

Various examples have been described. These and other examples are within the scope of the following numbered examples and claims.
Example 1: A device includes a glucose monitor includes cause delivery of a cleaning electrical current to the at least one sensor electrode, the cleaning electrical current being one or more electrochemical cleaning pulses configured to at least partially remove, in vivo and from the sensor electrode, excipients associated with the delivery of a fluid that includes insulin.
Example 2: The device of example 1, further comprising an infusion cannula configured to deliver insulin to the patient.
Example 3: The device of example 1 or example 2, wherein the one or more processors are further configured to determine an impedance at the at least one sensor or a signal current at the at least one sensor.
Example 4: The device of example 3, wherein the one or more processors are configured to cause delivery of the cleaning current responsive to a determination that the impedance at the glucose monitor satisfies an impedance threshold or a threshold rate of impedance change, or a signal electrical current from the glucose monitor satisfies a current threshold or satisfies a threshold rate of current change.
Example 5: The device of any of examples 1-3, wherein the one or more processors are configured to cause delivery of the cleaning current responsive to a user input to perform an electrochemical cleaning procedure.
Example 6: The device of example 3, wherein the one or more processors are configured to cause delivery of the cleaning current responsive to a determination that a time period has expired.
Example 7: The device of any of examples 1-6, wherein the cleaning electrical current is configured to be sourced from a working electrode and sunk at a counter electrode of the glucose monitor.
Example 8: The device of any of examples 1-6, wherein the cleaning electrical current is configured to be sourced from a counter electrode and sunk at a working electrode of the glucose monitor.
Example 9: The device of any of examples 1-6, wherein the cleaning electrical current is configured to be toggled between being sourced from a counter electrode of the glucose monitor and sunk at a working electrode of the glucose monitor, and sourced from the working electrode and sunk at the counter electrode.
Example 10: The device of any of examples 1-9, wherein the glucose monitor comprises a working electrode comprising an interference rejection membrane.
Example 11: The device of any of examples 1-10, wherein the glucose monitor comprises a working electrode comprising platinum.
Example 12: The device of any of examples 1-11, wherein the insulin comprises the excipients.
Example 13: The device of any of examples 1-12, wherein the excipients include one or more of phenol, m-cresol, or glycerin.
Example 14: The device of any of examples 1-12, wherein the one or more processors are configured to perform an initialization sequence, wherein the initialization sequence includes a plurality of pulses, each pulse of the plurality of pulses defining a voltage, current, and a duration, and wherein the one or more electrochemical cleaning pulses are substantially equal to at least one of the plurality of pulses in the initialization sequence in voltage, current or duration.
Example 15: The device of any of examples 1-14, wherein an electrochemical cleaning pulse of the one or more electrochemical cleaning pulses defines a segmented waveform, and wherein the segmented waveform includes a first segment, wherein the one or more processors are configured to detect an electroactive species based on the first segment, wherein the segmented waveform further includes a second segment configured to oxidize an analyte associated with the delivery of insulin through the infusion set, and a third segment configured to reduce an oxide layer formed during the oxide step to at least partially regenerate the glucose monitor.
Example 16: The device of any of examples 1-15, wherein an electrochemical cleaning pulse of the one or more electrochemical cleaning pulses defines a segmented waveform, and wherein a first segment of the segmented waveform defines a voltage amplitude of from about 500 millivolts to about 1500 millivolts.
Example 17: The device of any of examples 1-16, wherein the cleaning electrical current is a first cleaning electrical current, wherein the one or more processors are further configured to determine whether the first cleaning electrical current caused the signal current to match or fall within a threshold matching percentage of an expected signal current, and wherein, responsive to a determination that the signal current does not fall within the threshold matching percentage of the expected signal current, the one or more processors are further configured to cause delivery of a second cleaning electrical current.
Example 18: The device of any of examples 1-17, wherein the glucose monitor is configured to sense a change in impedance or signal current at the at least one sensor and determine that the change is due to delivery of a bolus dosage of insulin.
Example 19: The device of example 18, wherein the one or more processors is configured to cause delivery of the cleaning electrical current responsive to determining that a bolus dosage of insulin has been delivered.
Example 20: The device of example 19, where the one or more processors is configured to cause delivery of the cleaning electrical current after expiration of a time delay after determining that a bolus dosage of insulin has been delivered.
Example 21: A method includes determining, via one or processors implemented in circuitry and in communication with a memory of a device comprising a glucose monitor, that a cleaning electrical current should be delivered to at least one sensor electrode of the glucose monitor, wherein the at least one sensor electrode is configured to sense signals indicative of interstitial glucose level of a patient; and causing delivery of the cleaning electrical current to the at least one sensor electrode, the cleaning electrical current being one or more electrochemical cleaning pulses configured to at least partially remove, in vivo and from the sensor electrode, excipients associated with the delivery of a fluid that includes insulin.
Example 22: The method of example 21, further comprising a method for performing techniques of any of examples 2-20.
Example 23: A non-transitory computer-readable storage medium having stored thereon instructions that, when executed, configure a processor to: determine, via one or processors implemented in circuitry and in communication with a memory of a device comprising a glucose monitor, that a cleaning electrical current should be delivered to at least one sensor electrode of the glucose monitor, wherein the at least one sensor electrode is configured to sense signals indicative of interstitial glucose level of a patient; and cause delivery of the cleaning electrical current to the at least one sensor electrode, the cleaning electrical current being one or more electrochemical cleaning pulses configured to at least partially remove, in vivo and from the sensor electrode, excipients associated with the delivery of a fluid that includes insulin.
Example 24: The computer-readable storage medium of example 23, further comprising instructions that cause the one or more processors to perform techniques of any of examples 2-20.

Further disclosed herein is the subject-matter of the following clauses:
1. A device comprising:
   a glucose monitor comprising at least one sensor electrode configured to sense signals indicative of interstitial glucose level of a patient;
   a memory; and
   one or more processors implemented in circuitry and in communication with the memory, the one or more processors configured to:
      cause delivery of a cleaning electrical current to the at least one sensor electrode, the cleaning electrical current being one or more electrochemical cleaning pulses configured to at least partially remove, in vivo and from the sensor electrode, excipients associated with the delivery of a fluid that includes insulin.
2. The device of clause 1, further comprising an infusion cannula configured to deliver insulin to the patient.
3. The device of clause 1 or 2, wherein the one or more processors are further configured to determine an impedance at the at least one sensor or a signal current at the at least one sensor.
4. The device of clause 3 or of any of the clauses 1 to 3, wherein the one or more processors are configured to cause delivery of the cleaning current responsive to a determination that the impedance at the glucose monitor satisfies an impedance threshold or a threshold rate of impedance change, or a signal electrical current from the glucose monitor satisfies a current threshold or satisfies a threshold rate of current change.
5. The device of clause 1 or of any of the clauses 1 to 4, wherein the one or more processors are configured to cause delivery of the cleaning current responsive to a user input to perform an electrochemical cleaning procedure.
6. The device of clause 3 or of any of the clauses 1 to 5, wherein the one or more processors are configured to cause delivery of the cleaning current responsive to a determination that a time period has expired.
7. The device of clause 1 or of any of the clauses 1 to 6, wherein the cleaning electrical current is configured to be sourced from a working electrode and sunk at a counter electrode of the glucose monitor.
8. The device of clause 1 or of any of the clauses 1 to 7, wherein the cleaning electrical current is configured to be sourced from a counter electrode and sunk at a working electrode of the glucose monitor.
9. The device of clause 1 or of any of the clauses 1 to 8, wherein the cleaning electrical current is configured to be toggled between being sourced from a counter electrode of the glucose monitor and sunk at a working electrode of the glucose monitor, and sourced from the working electrode and sunk at the counter electrode.
10. The device of clause 1 or of any of the clauses 1 to 9, wherein the glucose monitor comprises a working electrode comprising an interference rejection membrane.
11. The device of clause 1 or of any of the clauses 1 to 10, wherein the glucose monitor comprises a working electrode comprising platinum.
12. The device of clause 1 or of any of the clauses 1 to 11, wherein the insulin comprises the excipients.
13. The device of clause 1 or of any of the clauses 1 to 12, wherein the excipients include one or more of phenol, m-cresol, or glycerin.
14. The device of clause 1 or of any of the clauses 1 to 13, wherein the one or more processors are configured to perform an initialization sequence,
   wherein the initialization sequence includes a plurality of pulses, each pulse of the plurality of pulses defining a voltage, current, and a duration, and
   wherein the one or more electrochemical cleaning pulses are substantially equal to at least one of the plurality of pulses in the initialization sequence in voltage, current or duration.
15. The device of clause 1 or of any of the clauses 1 to 14, wherein an electrochemical cleaning pulse of the one or more electrochemical cleaning pulses defines a segmented waveform, and
   wherein the segmented waveform includes a first segment, wherein the one or more processors are configured to detect an electroactive species based on the first segment, wherein the segmented waveform further includes a second segment configured to oxidize an analyte associated with the delivery of insulin through the infusion set, and a third segment configured to reduce an oxide layer formed during the oxide step to at least partially regenerate the glucose monitor.
16. The device of clause 1 or of any of the clauses 1 to 15, wherein an electrochemical cleaning pulse of the one or more electrochemical cleaning pulses defines a segmented waveform, and
   wherein a first segment of the segmented waveform defines a voltage amplitude of from about 500 millivolts to about 1500 millivolts.
17. The device of clause 1 or of any of the clauses 1 to 16, wherein the cleaning electrical current is a first cleaning electrical current,
   wherein the one or more processors are further configured to determine whether the first cleaning electrical current caused the signal current to match or fall within a threshold matching percentage of an expected signal current, and
   wherein, responsive to a determination that the signal current does not fall within the threshold matching percentage of the expected signal current, the one or more processors are further configured to cause delivery of a second cleaning electrical current.
18. The device of clause 1 or of any of the clauses 1 to 17, wherein the glucose monitor is configured to sense a change in impedance or signal current at the at least one sensor and determine that the change is due to delivery of a bolus dosage of insulin.
19. A method comprising:
   determining, via one or processors implemented in circuitry and in communication with a memory of a device comprising a glucose monitor, that a cleaning electrical current should be delivered to at least one sensor electrode of the glucose monitor, wherein the at least one sensor electrode is configured to sense signals indicative of interstitial glucose level of a patient; and
   causing delivery of the cleaning electrical current to the at least one sensor electrode, the cleaning electrical current being one or more electrochemical cleaning pulses configured to at least partially remove, in vivo and from the sensor electrode, excipients associated with the delivery of a fluid that includes insulin.
20. A non-transitory computer-readable storage medium having stored thereon instructions that, when executed, configure a processor to:
   determine, via one or processors implemented in circuitry and in communication with a memory of a device comprising a glucose monitor, that a cleaning electrical current should be delivered to at least one sensor electrode of the glucose monitor, wherein the at least one sensor electrode is configured to sense signals indicative of interstitial glucose level of a patient; and
   cause delivery of the cleaning electrical current to the at least one sensor electrode, the cleaning electrical current being one or more electrochemical cleaning pulses configured to at least partially remove, in vivo and from the sensor electrode, excipients associated with the delivery of a fluid that includes insulin.

## Claims

1. A device comprising:
a glucose monitor comprising at least one sensor electrode configured to sense signals indicative of interstitial glucose level of a patient;
a memory; and
one or more processors implemented in circuitry and in communication with the memory, the one or more processors configured to:
cause delivery of a cleaning electrical current to the at least one sensor electrode, the cleaning electrical current being one or more electrochemical cleaning pulses configured to at least partially remove, in vivo and from the sensor electrode, excipients associated with the delivery of a fluid that includes insulin.

2. The device of claim 1, further comprising an infusion cannula configured to deliver insulin to the patient.

3. The device of claim 1 or 2, wherein the one or more processors are further configured to determine an impedance at the at least one sensor or a signal current at the at least one sensor.

4. The device of any of the claims 1 to 3, wherein the one or more processors are configured to cause delivery of the cleaning current responsive to a determination that the impedance at the glucose monitor satisfies an impedance threshold or a threshold rate of impedance change, or a signal electrical current from the glucose monitor satisfies a current threshold or satisfies a threshold rate of current change.

5. The device of any of the claims 1 to 4, wherein the one or more processors are configured to cause delivery of the cleaning current responsive to a user input to perform an electrochemical cleaning procedure,
and/or
wherein the one or more processors are configured to cause delivery of the cleaning current responsive to a determination that a time period has expired.

6. The device of any of the claims 1 to 5, wherein the cleaning electrical current is configured to be sourced from a working electrode and sunk at a counter electrode of the glucose monitor,
and/or
wherein the cleaning electrical current is configured to be sourced from a counter electrode and sunk at a working electrode of the glucose monitor.

7. The device of any of the claims 1 to 6, wherein the cleaning electrical current is configured to be toggled between being sourced from a counter electrode of the glucose monitor and sunk at a working electrode of the glucose monitor, and sourced from the working electrode and sunk at the counter electrode.

8. The device of any of the claims 1 to 7, wherein the glucose monitor comprises a working electrode comprising an interference rejection membrane,
and/or
wherein the glucose monitor comprises a working electrode comprising platinum,
and/or
wherein the glucose monitor is configured to sense a change in impedance or signal current at the at least one sensor and determine that the change is due to delivery of a bolus dosage of insulin.

9. The device of any of the claims 1 to 8, wherein the insulin comprises the excipients,
and/or
wherein the excipients include one or more of phenol, m-cresol, or glycerin.

10. The device of any of the claims 1 to 9, wherein the one or more processors are configured to perform an initialization sequence,
wherein the initialization sequence includes a plurality of pulses, each pulse of the plurality of pulses defining a voltage, current, and a duration, and
wherein the one or more electrochemical cleaning pulses are substantially equal to at least one of the plurality of pulses in the initialization sequence in voltage, current or duration.

11. The device of any of the claims 1 to 10, wherein an electrochemical cleaning pulse of the one or more electrochemical cleaning pulses defines a segmented waveform, and
wherein the segmented waveform includes a first segment, wherein the one or more processors are configured to detect an electroactive species based on the first segment, wherein the segmented waveform further includes a second segment configured to oxidize an analyte associated with the delivery of insulin through the infusion set, and a third segment configured to reduce an oxide layer formed during the oxide step to at least partially regenerate the glucose monitor.

12. The device of any of the claims 1 to 11, wherein an electrochemical cleaning pulse of the one or more electrochemical cleaning pulses defines a segmented waveform, and
wherein a first segment of the segmented waveform defines a voltage amplitude of from about 500 millivolts to about 1500 millivolts.

13. The device of any of the claims 1 to 12, wherein the cleaning electrical current is a first cleaning electrical current,
wherein the one or more processors are further configured to determine whether the first cleaning electrical current caused the signal current to match or fall within a threshold matching percentage of an expected signal current, and
wherein, responsive to a determination that the signal current does not fall within the threshold matching percentage of the expected signal current, the one or more processors are further configured to cause delivery of a second cleaning electrical current.

14. A method comprising:
determining, via one or processors implemented in circuitry and in communication with a memory of a device comprising a glucose monitor, that a cleaning electrical current should be delivered to at least one sensor electrode of the glucose monitor, wherein the at least one sensor electrode is configured to sense signals indicative of interstitial glucose level of a patient; and
causing delivery of the cleaning electrical current to the at least one sensor electrode, the cleaning electrical current being one or more electrochemical cleaning pulses configured to at least partially remove, in vivo and from the sensor electrode, excipients associated with the delivery of a fluid that includes insulin.

15. A non-transitory computer-readable storage medium having stored thereon instructions that, when executed, configure a processor to:
determine, via one or processors implemented in circuitry and in communication with a memory of a device comprising a glucose monitor, that a cleaning electrical current should be delivered to at least one sensor electrode of the glucose monitor, wherein the at least one sensor electrode is configured to sense signals indicative of interstitial glucose level of a patient; and
cause delivery of the cleaning electrical current to the at least one sensor electrode, the cleaning electrical current being one or more electrochemical cleaning pulses configured to at least partially remove, in vivo and from the sensor electrode, excipients associated with the delivery of a fluid that includes insulin.
